# EUROPEAN PATENT SPECIFICATION

(11) **EP 1 354 210 B1**
(45) Date of publication and mention of the grant of the patent: **15.11.2006**
(21) Application number: 02716509.1
(22) Date of filing: 24.01.2002
(51) Int. Cl.: G01N 33/68

(54) **METHOD FOR CANCER DIAGNOSTICS**
KREBSDIAGNOSEVERFAHREN
PROCEDE DE DIAGNOSTIC DU CANCER

(30) Priority: 24.01.2001 SE 0100197
(43) Date of publication of application: 22.10.2003
(73) Proprietor: Erlandsson, Fredrik, 115 23 Stockholm (SE); Zetterberg, Anders, 182 61 Djursholm (SE)
(72) Inventor: Erlandsson, Fredrik, 115 23 Stockholm (SE); Zetterberg, Anders, 182 61 Djursholm (SE)
(74) Representative: Hyltner, Jan-Olof
(86) International application number: PCT/SE2002/000116
(87) International publication number: WO 2002/059616

(56) References cited:
- WO-A1-94/17414
- FREDRIK ERLANDSSON ET AL.: 'A detailed analysis of cyclin a accumulation at the G1/S border in normal and transformed cells' EXPERIMENTAL CELL RESEARCH vol. 259, 2000, pages 86 - 95, XP002950266
- DUTTA A. ET AL.: 'Cyclins as markers of tumor priliferation: immunocytochemical studies in breast cancer' PROC NATL ACID SCI vol. 92, no. 12, June 1995, pages 5386 - 5390, XP002950267
- KHANDAN KEYOMARSI ET AL.: 'Redundant cyclin overexpression and gene amplification in breast cancer cells' PROC. NATL. ACAD. SCI. vol. 90, February 1993, pages 1112 - 1116, XP002950268
- JIANPING GONG ET AL.: 'Unscheduled expression of cyclin B1 and cyclin E in several leukemic and solid tumor cell lines 1' CANCER RESEARCH vol. 54, August 1994, pages 4285 - 4288, XP002950269

## Description

### TECHNICAL AREA

The presented invention is a method to diagnose cancer and pre-cancerous lesions in tissue and cell samples, and to gain prognostic and predictive information when a cancer disease has already been diagnosed.

### CURRENT TECHNICAL STANDPOINT

Routine cancer diagnostics is based on microscopic analysis of cell and tissue samples from tumours or tumour suspected tissues. The tissue is stained so that individual cells and groups of cells can be identified. The routine diagnosis is based on morphological properties, i e alterations in shape, size, and staining characteristics of the cells, and on irregularities in tissue architecture. Cancer diagnostics is thus based on a subjective evaluation of morphologic deviation from the corresponding normal tissue.

Long training and much experience is required to achieve the skill to make the correct diagnosis with sufficient certainty. Borderline cases between cancer and pre-cancerous lesions, and between pre-cancerous and non-cancerous lesions, can present a challenge to even the most experienced pathologists or cytologists. The problem is increasing as many tumours today are detected early, and therefore have not yet fully developed the morphologic criteria for cancer. Another aspect of morphological analysis is the evaluation of the degree of malignancy of the tumour (tumour grade), on which the choice of tumour therapy is often based. This is a problem since in many cases the morphology of the tumour does not reflect the true malignancy of the tumour. With this in mind it is obvious that new procedures to diagnose and evaluate malignancy in an objective and quantitative way would be a major breakthrough.

The transition from normal cell to cancer cell is generally called transformation and is due to the sequential alteration of several specific genes in a normal cell. During transformation the cell acquires the properties of a cancer cell, namely to invade surrounding tissue and form daughter tumours (metastasise). Major breakthroughs have been made in experimental cancer research within the last two decades. Around 50 controlling genes have been identified which can be altered in cancer cells. Some of these genes are hyper active in the cancer cell (oncogenes), which results in an abundance of signals for cell division. Other controlling genes are often inactivated in cancer cells (tumour suppressor genes). In normal cells tumour suppressor genes commonly balance the growth stimulatory signals from oncogenes. No single entirely cancer specific genetic alteration has yet been identified. We have therefore focused on finding a combination of properties, where the combination itself is abnormal in the cancer cell.

Important breakthroughs have been made during the last decade in cell cycle research, i e research on the co-ordination between replication of the genome and the cell division. The central components of the cell cycle machinery have been identified, and were found to be conserved during the last one thousand million years of evolution. The components are of universal importance in yeast, plants and animals. A general picture of the processes involved in cell cycle control has been generated. Two principally different biochemical processes regulate the cell cycle. One is reversible phosphorylation, i e phosphate groups beeing bound to or removed from target proteins, thereby changing the structure and function of these proteins. The process is controlled by proteins called kinases. The major kinases in cell cycle regulation are the so-called cyclin dependent kinases (CDKs), whose activity is regulated by the cyclin proteins. The second process is the highly regulated synthesis and degradation of the cyclin proteins. Two cyclins relevant to this application are cyclin E and cyclin A.

It has previously been assumed that the cell cycle operates in the same way in cancer cells and normal cells, and that it is mainly the proliferation control mechanism (the transmission of growth regulatory signals from the outside of the cell to the genome in the cell nucleus) that is defective. The surprising discovery that the cell cycle itself is altered in cancer has quickly become of central importance for cancer research, and is a possible cause for the chromosomal instability seen in cancer cells. Thus both the uncontrolled proliferation and the chromosomal instability of the tumour cell seem to be caused by defective cell cycle regulation. This new knowledge about the cell cycle and the defective cell cycle control in tumour cells has the potential to be utilised in future cancer diagnostics.

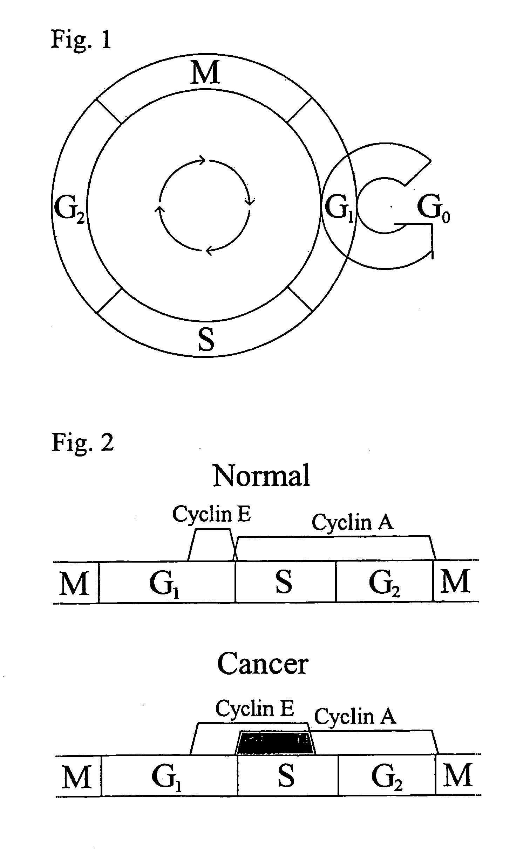

The cell cycle is divided in different phases based on the replication of the genome and the division of the cell. The cell division phase, during which the chromosomes are divided between the two new daughter cells, is called the M-phase (mitosis). Between each M-phase the cell copies its genome through DNA synthesis (also called DNA replication). The phase during which the DNA is copied is called the S-phase (DNA synthesis phase). Between the M-phase and the S-phase, two other phases can be identified. The first of these, i e the gap between the M-phase and the S-phase, is called G₁. The second gap, i e the gap between the S-phase and the M-phase, is called G₂. The complete cell cycle thus includes the phases M - G₁ - S - G₂ - M, see fig 1. A cell that has recently been born at the division starts its new cell cycle in G₁. If it decides to divide again it proceeds through G₁ and enters the S-phase, during which it copies its DNA. When a complete copy of the genome has been synthesised the cell progresses to G₂, during which it prepares for mitosis (M-phase). The cell then continues into M-phase, during which the chromosomes are separated to each new daughter cell formed by cell division. The daughter cells are now back in G₁, and the cell cycle is completed. Both normal and cancer cells go through the phases of the cell cycle described above.

The majority of the cells in multi-cellular organisms, e g in man, is in a quiescent (resting) state called G₀. Cells can remain in G₀ for a long time, and they enter G₁ in response to growth stimulatory signals. In some tissues practically all cells are resting in G₀, e g in muscle or nervous tissue. Other tissues, e g intestine, skin, bone marrow, embryonic tissue, and tumour tissue, contain both cells in G₀ and in the cell cycle (phases G₁, S, G₂ and M).

Much effort has been spent to find out which factors and processes control the progression from one phase of the cell cycle to the next, such as the transition from G₁ to S-phase. Proteins have been identified which only are present at specific phases of the cell cycle. One of these proteins is cyclin E, which was discovered in 1991 by Lew et al., see Lew, D. J., Dulic, V., Reed, S. I., Isolation of three novel human cyclins by rescue of G₁ cyclin (Cln) function in yeast, Cell 66, pages 1197 - 1206, 1991. The protein cyclin E is expressed in a cell cycle specific manner. Studies have shown that in normal cells cyclin E is present in the cell nucleus only during the last part of G₁ and the first part of S. Another related protein with a cell cycle specific expression is cyclin A, which appears when the cell enters S, and remains in the cell nucleus until M, see Pines, J., Hunter, T., Human cyclin A is adenovirus E1A-associated protein p60 and behaves differently from cyclin B, Nature 346, pages 760-763, 1990, and Erlandsson, F., Linnman, C., Ekholm, S., Bengtsson, E., Zetterberg, A., A detailed investigation of cyclin A accumulation at the G₁/S border in normal and transformed cells, Experimental Cell Research 259, pages 86 - 95, 2000.

Cyclin E has been shown to exist in abnormally high levels in some tumours, and tumour derived cell lines, see Keyomarsi, K., Pardee, A. B. Redundant cyclin overexpression and gene amplification in breast cancer cells, Proc Natl Acad Sci, USA 90, pages 1112 - 1116, 1993. The cyclin E level was measured in cells in culture synchronised at different stages of the cell cycle. Although the cell cycle is disturbed by the synchronisation the data indicate that cyclin E is expressed not only in G₁ in tumour cells in culture. The presented method can however not be used for studying the expression pattern of cyclin E over the cell cycle in tumour tissues. It can only be applied to experimental tumour cells grown in culture.

In 1994 Keyomarsi et al. showed that defective cyclin E molecules are present in tumours, and the presence of such defective cyclin E molecules may be an indication for poor prognosis, see Keyomarsi, K., O'Leary, N., Molnar, G., Lees, E., Fingert, H. J., Pardee, A. B., Cyclin E, a potential prognostic marker for breast cancer, Cancer Research 54, pages 380 - 385, 1994. In this study tissue biopsies from human cancer were investigated. Keyomarsi et al. found that tumour tissue contained more cyclin E than surrounding normal tissue. They also found defective forms of the cyclin E molecule in some tumours. The authors suggest a link between the tumour malignancy, and the levels of cyclin E and defective cyclin E in the tumour. However, by the method used it could not be determined if the increased levels of cyclin E was simply due to an increased number of cells in the cell cycle in the tumour, or if it was due to abnormal expression of cyclin E. The investigations were carried out by a biochemical-immunological method called "western blot". By this method a sample of suspected tumour tissue, which contains a mixture of proliferating and quiescent normal and cancer cells. The tissue sample is homogenised, and a protein mixture is then isolated from the sample. This protein mixture is then passed through a gel, which separates different proteins based on size and/or electrical charge. The protein of interest is subsequently marked using an antibody specific for the protein and labelled with a radioactive isotope or a colour. The main drawback of the method when applied to suspected tumour samples in a clinical setting is that it is not possible to tell if the detected cyclin E originates from the normal cells or the tumour cells in the tissue sample.

During the past five years several other studies have investigated the use of cyclin E levels in tissue samples as a diagnostic tool. The investigations are either carried out using western blot techniques, which as described above measures the total protein level in the sample, or based on immunohistochemical techniques, by which the frequency of cells containing cyclin E in the tumour tissue can be measured. The main drawback of the western blot technique is that it can not make a distinction whether a high level of cyclin E in the tumour sample is the result of an over expression or a cell cycle abnormality. Furthermore, it can not be used to detect a small number of cancer cells in a large population of normal cells, which often is the case in clinical tissue samples. In order to obtain a sample that only contains tumour cells some research groups have used micro dissection to obtain a partly purified sample containing mainly tumour cells. Micro dissection is however a very tedious procedure, and it is based on morphological diagnostic procedures. An efficient diagnostic technique must be possible to apply even to samples that are not already known to contain tumour cells. The different variations of the western blot technique are scientifically very interesting, but the procedures are generally very time consuming, and western blot is too blunt a tool to be possible to use in clinical diagnostics.

An alternative way to investigate the presence of cyclin E is by the use of immunohistochemical staining procedures. In this procedure the sample is incubated with antibodies against cyclin E which can be coloured. Subsequently the cells in the sample containing cyclin E can be detected using a microscope. An increased number of cells containing cyclin E in the sample could either represent an increased number of cells proliferating, i e being in the cell cycle, or reflect a cell cycle abnormality with respect to cyclin E expression, i e cyclin E expressed in other cell cycle phases than G₁. It is not possible to make a distinction between these two alternatives only by analysing cyclin E without knowing the cell cycle position of the cells containing cyclin E. Attempts have been made to get information about proliferation by staining a parallel sample for another cell cycle marker such as cyclin A (see Dutta, A., Chandra, R., Leiter, L. M., Lester, S. 1995 Cyclins as markers of tumour proliferation: Immunocytochemical studies in breast cancer. Proc Natl Acad Sci USA 92, pages 5386-5390). This may provide additional information about proliferative activity in the tumour, but information about cell cycle abnormalities in the expression pattern of cyclin E can only be obtained by combining the cyclin E staining with the staining for a cell cycle marker on the individual cells, as we propose below.

### SUMMARY OF THE INVENTION

The basis for the invention presented in this application is the fact that cyclin E is abnormally regulated in the cell cycle of the cancer cell, i e cyclin E is present in the wrong phases of the cell cycle. In normal cells cyclin E is only present in the cell nucleus during late G₁ and early S, while it appears throughout S, and even in G₂ in cancer cells. This has opened the possibility to develop a diagnostic procedure based on the abnormal presence of cyclin E in later stages of the cell cycle (late S and G₂) in cancer cells.

The invention therefore relates to a method for analysing cancer disease wherein the presence of one or more proteins of cyclin E type and post G₁-substances selected from cyclin A, PCNA and bromo deoxyuridine, in one and the same cell is an indication of a cancer related disease. The invention also relates to a method of evaluating the degree of malignancy by detecting the amount of cells that contain both cyclin E type protein and post G₁-substances.

### DESCRIPTION OF THE INVENTION

One aspect of the invention is a procedure by which it is possible to diagnose cancer in a tissue sample by determining in individual cells whether there is an abnormal cell cycle expression of "cyclin E-type-protein". This means that "cyclin E-type-protein" is present in the latter part of the cell cycle, i e that "cyclin E-type-protein" remains throughout the majority of S and / or is present during G₂. The method is thus based on the knowledge that normal cells degrade their "cyclin E-type-protein" in early S, and that only cancer cell nuclei contain "cyclin E-type-protein" throughout S, and sometimes even G₂. The method is a combination of two measurements done on the same cell by for example immunohistochemical technique, a measurement of the level of "cyclin E-type-protein" in individual cells is combined with a determination of the position in the cell cycle (G₁, S, or G₂) for each of the investigated cells. If "cyclin E-type-proteins" appear in cells in late S and / or G₂ in an elevated number of cells in the investigated cell or tissue sample, then there are cancer cells. Information regarding the percentage of cells in late S or G₂ that contains "cyclin E-type-proteins" is not only of use for making an accurate diagnosis, it also has prognostic value, i e it can provide information of how malignant the tumour cells are. This is true since it is likely that a tumour cell population with more cells with disturbed cell cycle regulation is more malignant than a tumour cell population with less disturbed cell cycle control. Knowledge of the degree of malignancy of the individual tumour is very important for the selection of type of therapy.

Throughout this document "cyclin E-type-protein" is defined as the cyclin E protein (both defective and normal cyclin E molecules), as well as other proteins, which similarly to the cyclin E protein is removed from the cell nucleus in early S, but remains longer in cancer cells. Thus all "cyclin E-type proteins" are present in the cell nucleus of the normal cell only during G₁ and the first part of S. Afforementioned "cyclin E-type-proteins" mainly include the two isoforms of cyclin E, called cyclin E1 and cyclin E2. The expression pattern of cyclin E in normal and tumour cells are shown in fig. 2. Other examples of such "cyclin E-type-proteins" are the mutated forms of cyclin E with molecular weights of 42 and 35 kDa described by Keyomarsi, as mentioned above, and other proteins not related to cyclin E, but with a similar expression pattern as cyclin E in normal and cancer cells.

The level of "cyclin E-type-protein" is measured in each individual cell preferably by immunohistochemic technique. See Brandtzaeg, P., Halstensen, T. S., Huitfeldt, H. S., and Valnes, K.N. (1997) Immunohistochemistry: A practical approach 2. Editors Johnstone, A. P. and Turner, M. W., IRL, Oxford, pages 71-130, for an excellent review of many of the various immunohistochemic methods available. Individual cells containing "cyclin E-type-protein" can be detected in both tissue samples and cells in suspension by using a microscope or a flow cytometer. The expression pattern of "cyclin E-type-protein" during the cell cycle can theoretically be done in many different ways. In Gong, J. et al. (1994) Cancer Research 54 (16), pages 4285-4288, the amount of DNA in each cell is used to determine the location in the cell cycle in combination with determination of cyclin E levels. A severely limiting factor in their procedure is however that the amount of DNA in the cell only can be used as a marker for position in the cell cycle when strictly diploid cells analysed, i e on cells with 46 chromosomes where a G₁ cell has 2c relative DNA units (approximately 6 pg of DNA) and a G₂ cell has 4c relative units of DNA. Cells in S then have between 2c and 4c relative units of DNA, as they are under way to copy their DNA. The problem is that tumour cells very often are aneuploid, i e they do not have exactly 46 chromosomes, and therefor do not contain exactly 2c DNA in G₁. Furthermore does the degree of aneuploidy in a tumour cell population frequently vary considerably, and the cells in G₁ can have DNA contents ranging from 1.5c to >6c in one single tumour (see for example Forsslund et al., Cancer, Oct 15 1996, 78(8), pages 1748-55 or Auer G. et al., Anal Quant Cytol Histol, May 1987; 9(2), pages 138-46). This naturally makes it impossible to apply the method described by Gong et al. to the vast majority of human tumours, and is the main reason why the work presented by Gong et al. is not relevant to this application. We have instead developed a method to determine cell cycle position that is independent of DNA content. Our method is based on analysis of the content of a cell cycle specific marker by staining for a substance which is only present during S and/or G₂ in cancer cells. Proteins that are only present in the cell or cell nucleus exclusively during S and/or G₂ are herein called "post G₁-substances". "Post G₁-substances" used according to the invention are cyclin A, PCNA and bromodeoxyuridine (BrdU) in BrdU-incorporated cell populations.

Simultaneously staining for "cyclin E-type-protein" and "post G₁-substance" allows image cytometric or flow cytometric measurements of the levels of "cyclin E-typ-protein" and "post G₁-substance" in the same cell nuclei. The images or flow cytometric measurements can be analysed using many principally different methods. The images can for example be segmented by simple grey level thresholding, maximum likelihood classification, or watershed algorithms. See Gonzales, R C., Woods, R. E., 1993, *Digital Image Processing,* Addison-Wesley, New York, chapter 7, for a review of most of the currently available methods for image segmentation. Classification of the investigated cells into cells staining positive or negative with respect to each stain can also be performed using a wide variety of readily available methods, such as Bayes classification, neural network based classification, the classification presented by us in Erlandsson, F., Linnman, C., Ekholm, S., Bengtsson, E., Zetterberg, A., 2000, Exp Cell Res 259, pages 86-95, or any other reliable method by which the negative and positive cells can be divided. See Gonzales, R. C., Woods, R. E., 1993, *Digital Image Processing,* Addison-Wesley, New York, chapter 9, for a review of some of the best known methods for classification. Furthermore, classification is not always necessary, instead the actual measured values representing the staining intensity in each cell can be directly used in the statistical analysis. Calculating the correlation between the measured level of "cyclin E-type-protein" and "post G₁-substance" will for example make it possible to distinguish normal cell populations from cancer cell populations, and less malignant cell populations from more malignant cell populations. Flow cytometers often come equipped with software containing appropriate algorithms for the analysis of the measured staining intensities. Finally can the evaluation of the stained samples be done manually by an observer who simply counts the cells, and subjectively decides by visual evaluation whether a cell contains "cyclin E-type-protein" and/or "post G₁-substance".

By statistic analysis of whether "cyclin E-type-protein" and "post G₁-substance" appear in an increased percentage of the cells in the population it can be decided whether there are cancer cells in the investigated sample, and how malignant the cancer cells are. The percentage of cells in S or G₂ containing cyclin E is high in highly malignant cancer cell populations and low in normal cell populations, in general more than 40% in highly malignant tumours, and less than 10% in normal tissue. The exact percentage varies with tumour type, sampling procedure, and staining and analysis procedure used. These variables must be established in advance, before the method can be routinely used. A major advantage of the invention is that it represents a method that is independent of the proliferation in the investigated tissue. The method is instead depending on the percentage of cells that are simultaneously positive for "cyclin E-type-protein" and "post G₁-substance", and therefore measures the presence of cells with abnormally regulated cell cycle.

A major advantage with our method is that it allows an objective and quantitative determination of whether cancer is present or not. Therefore the method can be developed to an automated and fast cancer test with high capacity. Yet another advantage is the high sensitivity of the method, i e it is sufficient to detect even a very small number of cells in S with "cyclin E-type-protein". Furthermore, the sample is not destroyed during the investigation when it is carried out as described herein, which makes it possible to follow up cases of special interest with a traditional microscopic investigation, during which the cells in S or G₂ containing "cyclin E-type-protein" can be examined. These properties, suitability for automation and high sensitivity, make the method ideal for screening purposes. One example is cervical smear evaluations, in which a small number of abnormal cells have to be detected.

### DESCRIPTION OF FIGURES

Figure 1 displays a schematic representation of the cell cycle and its phases.
Figure 2 shows the expression pattern of cyclin E and cyclin A during the cell cycle in a normal cell (upper) and in a cancer cell (lower). Note that cyclin E and cyclin A are expressed in sequence in the normal cell, whereas the cyclin E and cyclin A expression patterns are overlapping, i e cyclin E and cyclin A are expressed simultaneously during S-phase in the cancer cell.
Figure 3 displays the distribution of cyclin A and cyclin E expression in a normal cervical epithelium. Each dot represents one individual cell. Compare with figure 2.
Figure 4 displays the distribution of cyclin A and cyclin E expression in a less malignant cervical carcinoma tumour, as the patient is still alive and well 6 years after treatment. Each dot represents one individual cell. Compare with figures 2 and 3.
Figure 5 displays the distribution of cyclin A and cyclin E expression in a highly malignant cervical carcinoma tumour, as the patient died within 3 years after primary treatment. Each dot represents one individual cell. Compare with figures 2, 3 and 4.

### DESCRIPTION OF AN APPLICATION

Below are a couple of examples of the method described in accordance with the invention, and supported by the figures. In the examples below cyclin E is used as an example of a "cyclin E-type-protein", and cyclin A is used as an example of a "post G₁-substance".

The task consists of using the method according to the invention to determine the cyclin E level in the individual investigated cells, while also determining the position in the cell cycle of each investigated individual cell. In one example an immunohistochemical double staining technique is used for staining the cells in from cervical carcinoma biopsies acquired from patients prior to treatment. There are no major technical differences between performing the procedure on cells in a monolayer culture, on cells in a cytologic sample, or on cells in a sectioned tissue sample. Routinely handled, i e formaldehyde fixed and paraffin embedded, tissue sections from patients with cervical carcinoma were studied in order to perform an investigation of the expression pattern of cyclin E *in vivo.* The tissue sections were cut at a thickness of 0.4µm. The sections were incubated overnight at 47°C to adhere to Superfrost Plus microscope slides from Menzler Gläser. The sections were stored at -20°C and then stepwise deparaffinized in graded alcohols prior to staining. Antigenic recovery was performed by cooking twice the sections for 5 minutes in a citrate buffer at pH 6.0 using a microwave oven.

The tissue sections were stained using the cyclin E monoclonal antibody (HE12) and a rabbit polyclonal antibody directed against cyclin A (H-432) from Santa Cruz Biotechnology. The secondary antibodies used included a FITC-conjugated anti-rabbit antibody, and a Cy3-conjugated anti-mouse antibody from Jackson ImmunoResearch. The following steps were all executed at room temperature unless stated otherwise. Prior to staining the slides were washed in washing buffer (0.3 mM NaCl and 0.02% Tween 20 in a buffer consisting of 0.05mM Tris-HCl at pH 7.6) for 10 minutes, followed by an incubation for 15 minutes in blocking buffer (1% bovine serum albumin and 0.5% Tween 20 dissolved in PBS) to block non-specific binding of the primary antibodies. Thereafter they were incubated with the primary antibodies diluted in blocking buffer for 48 hours at 4° C. Unbound and non-specifically bound antibodies were removed by extensive washing in washing buffer for 3 times 15 minutes.

To block non-specific binding of the secondary antibodies the cover microscope slides were incubated with 4% donkey serum diluted in blocking buffer for 30 minutes. The secondary antibodies, diluted in 4% donkey serum, were added during an incubation for 30 minutes at room temperature. The microscope slides were then washed in washing buffer for 3 times 15 minutes.

Finally the microscope slides were mounted for fluorescence microscopy in Vectashield mounting medium containing DAPI (4',6-diamidino-2-phenylindole, H-1200 from Vector Laboratories Inc). DAPI binds to DNA and allows the identification of individual cell nuclei in the sample. This brought the final number of fluorophores used in the experiment to three: FITC for cyclin A, Cy-3 for cyclin E, and DAPI for DNA.

When each fluorophore is illuminated, or excited, using light of a certain wavelength, it responds by emitting light, fluoresce, in another specific wavelength. It is possible to measure the level of fluorescence emitted by each fluorophore using a microscope equipped with interchangeable excitation and emission light filters and a camera. Thus a semiquantitative measurement of the concentration of cyclin E and cyclin A in each cell nucleus can be calculated.

All stained microscope slides were accompanied by a negative control consisting of an identical microscope slide with respect to cell type, fixation and storage time. The negative controls went through the same staining procedure, only excluding the primary antibodies and using blocking buffer instead. All negative controls exhibited a very low level of non-specific nuclear staining as compared to the stained microscope slides.

Images of the tumours were obtained using a Zeiss Plan-Neofluar 63x oil immersion lens on a Delta Vision system, produced by Applied Precision Inc, Issaquah, WA. The system consists of a mercury lamp with a fibre optic illumination system, conventional microscope optics, selective filters for excitation and emission, and a cooled CCD camera from Photometrics Ltd, Tucson, AZ. The acquired images had a resolution of 0.2µm. Image segmentation and data extraction was performed using the IMP image processing software, and the staining intensity measurements were analysed using the Matlab or Excel software packages. Between 800 and 3000 cells were analysed from each sample.

The image analysis started out with a subtraction of the background fluorescence in the images. Then the DAPI images were used to perform a segmentation, during which every individual cell nucleus in the images were defined. The masks created during the segmentation were then applied to the FITC (cyclin A) and Cy-3 (cyclin E) images, and thereby the fluorescence emitted by each of these fluorophores could be calculated for every individual cell. Note that the DNA stain DAPI was exclusively used to define the cell nuclei, and was not involved in the determination of the position in the cell cycle.

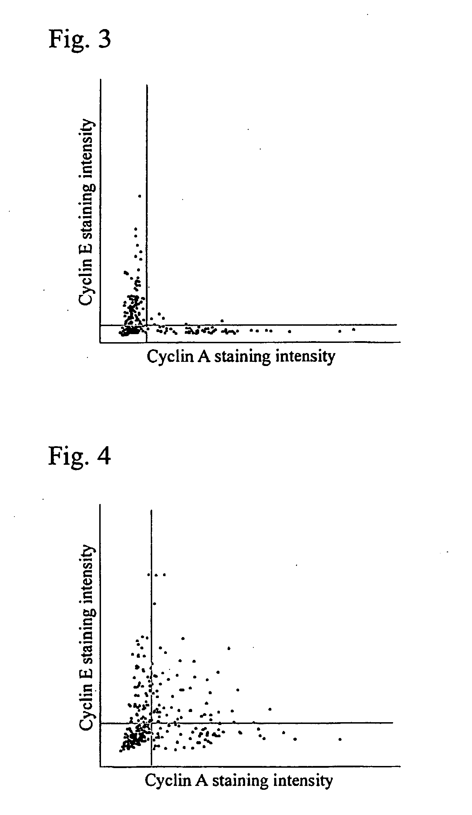

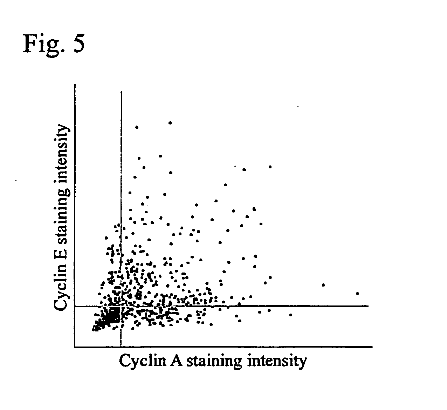

Now we could determine which of the investigated cells that were in S or G₂ by using cyclin A content as a marker for such cells. Figure 3, 4, and 5 display the main results. The diagrams show the distribution of cyclin A and cyclin E from three different tissue samples. Figure 3 shows normal cervical epithelium, figure 4 shows a tumour of low malignancy grade (the patient is still alive 6 years after treatment), and figure 5 shows a tumour of high malignancy grade (the patient died within 3 years after treatment).

The diagrams displayed in figures 3-5 clearly show the difference in how the cyclin E and cyclin A levels are related in each of the three cases. In cervical carcinomas cells with a high cyclin A content (i e cells in S or G₂) clearly contain more cyclin E than they do in normal cervical epithelium. The more malignant tumour cells show an even more abnormal cyclin E expression pattern. A very high percentage of cells in S and G₂ contain high levels of cyclin E. The presented procedure, which makes it possible to clearly detect differences in expression pattern of cyclin E over the cell cycle, is the essence of this invention.

The presented method has the potential to quickly become very useful in routine diagnostics in the near future since the above described abnormality is so obvious in cancer cells, and since the method presented is very simple to implement. If the method is further refined it may be possible to detect one single or a just a few cancer cells in a cell population consisting of several millions of cells, since cells containing both high levels of cyclin E and high levels of cyclin A simply does not seem to exist in normal cell populations. The method may also prove to be highly useful for determinations of the degree of malignancy of tumours. The method is very easy to automate, and can easily be combined with traditional diagnostic techniques, since the samples can be stained using for example the traditional HTX-eosine stain after being subjected to an evaluation according to the invention. The pathologist or cytologist investigating a sample which has been handled in such a manner can choose to focus his or her attention to the cells exhibiting the detected cell cycle abnormality.

## Claims

1. A method for detecting a disturbance of the cell cycle regulation in individual cells for diagnosing cancer or precancerous lesions in a cell or tissue sample or for prognostication of a determined cancer disease, **characterised in that** it comprises the steps of:
- detecting cyclin E in separate cells of the sample
- detecting post G₁-substances selected from cyclin A, PCNA and bromodeoxyuridine in separate cells of the sample
- identifying cells having cyclin E in the cell nucleus
- identifying cells being in S-phase or G₂-phase based on their content of the post G₁-substances and
- whereby an increased amount of cells having an increased content of cyclin E in the cell nucleus at the same time as the same cells are in the S-phase or G₂-phase is an indication of the presence in the sample of cells having a disturbed cell cycle regulation, which is of diagnostic and prognostic value in cancer diseases.

2. A method according to claim 1, **characterised in that** the detection of cyclin E is made by staining of the protein.

3. A method according to claim 2, **characterised in that** the staining of a chosen cyclin E is made by an antibody directed to the chosen cyclin E.

4. A method according to claim 1, **characterised in that** the determination of content of the post G₁-substance is made by staining thereof.

5. A method according to claim 4, **characterised in that** the staining of a chosen post G₁-substance is made by an antibody directed against the selected post G₁₋substance.

6. A method according to claim 5, **characterised in that** cyclin A is chosen as post G₁₋substance.

7. A method according to claim 2, **characterised in that** cells that are stained for
a) the content of cyclin E and
b) the content of post G₁-substance
are stained using two different colours, one specific for a) and the other specific for b).

8. A method according to claim 7, **characterised in that** the cells are illuminated and cells of type a) and b) are then identified **in that** each cell type respectively emits or absorbs light of a typical and specific wavelength.

9. A method according to claim 8, **characterised in that** an indication is obtained of the amount of cyclin E and post G₁-substance in each individual cell by analysing the light intensity or light absorbtion corresponding to cells coloured for a) and b) respectively.

10. A method according to claim 7, **characterised in that** the nucleus of the cells are identified by staining the sample with a colour that is specific for the cell nucleus, whereby the cell nucleus when illuminated emits or alternatively absorb light of a wavelength specific for the colour used.

11. A method according to claim 10, **characterised in that** the light emitted or absorbed by the sample is photographed or detected by a CCD camera with a filter adapted to separate the light from the cell nucleus and the light from the cells stained for a) and b), data related to the light intensity of each light wavelength from each cell nucleus is extracted in a computer program for image analysis, giving a measurement of both the content of cyclin E and post G₁-substance in each cell nucleus.

12. A method according to claim 10, **characterised in that** flow cytometric analysis is made of the sample giving the content of cyclin E and post G₁-substance in each cell nucleus.

13. A method according to claim 11 or 12, **characterised in that** information that a sample contains an increased amount of cyclin E in the S-phase and/or G₂-phase is an indication that the sample contains cells with a disturbed regulation of the cell cycle, which is of diagnostic and prognostic value in cancer diseases.

## Patentansprüche

1. Verfahren zum Nachweis einer Störung der Zellzyklusregulation bei individuellen Zellen zur Diagnose von Krebs oder präkanzerösen Läsionen in einer Zell- oder Gewebeprobe oder zum Prognostizieren einer festgestellten Krebserkrankung, **dadurch gekennzeichnet, dass** es die Schritte umfasst:
- Nachweis von Cyclin E in separaten Zellen der Probe;
- Nachweis von Post-G₁-Substanzen, ausgewählt aus Cyclin A, PCNA und Bromdesoxyuridin in separaten Zellen der Probe;
- Identifizierung von Zellen, die Cyclin E im Zellkern ausweisen;
- Identifizierung von Zellen, die sich in der S-Phase oder G₂-Phase befinden auf Basis ihres Gehalts an Post-G₁-Substanzen; und
- wobei eine erhöhte Menge von Zellen, die zu dem Zeitpunkt, an dem sich diese Zellen in der S-Phase oder der G₂-Phase befinden, einen erhöhten Cyclin E-Gehalt im Zellkern aufweisen, ein Anzeichen dafür ist, dass in der Probe Zellen mit einer gestörten Zellzyklusregulation vorliegen, was bei Krebserkrankungen von diagnostischem und prognostischem Wert ist.

2. Verfahren nach Anspruch 1, **dadurch gekennzeichnet, dass** der Nachweis von Cyclin E durch Färben des Proteins erfolgt.

3. Verfahren nach Anspruch 2, **dadurch gekennzeichnet, dass** das Färben eines gewählten Cyclin E mit einem Antikörper erfolgt, der gegen das gewählte Cyclin E gerichtet ist.

4. Verfahren nach Anspruch 1, **dadurch gekennzeichnet, dass** die Bestimmung des Gehalts an der Post-G₁-Substanz durch deren Färbung erfolgt.

5. Verfahren nach Anspruch 4, **dadurch gekennzeichnet, dass** das Färben einer gewählten Post-G₁-Substanz mit einem Antikörper erfolgt, der gegen die gewählte Post-G₁-Substanz gerichtet ist.

6. Verfahren nach Anspruch 5, **dadurch gekennzeichnet, dass** Cyclin A als Post-G₁-Substanz gewählt wird.

7. Verfahren nach Anspruch 2, **dadurch gekennzeichnet, dass** Zellen, die auf
a) den Cyclin E-Gehalt und
b) den Gehalt an Post-G₁-Substanz
gefärbt werden, mit zwei verschiedenen Farbstoffen gefärbt werden, einem, der für a) spezifisch ist und einem, der für b) spezifisch ist.

8. Verfahren nach Anspruch 7, **dadurch gekennzeichnet, dass** die Zellen bestrahlt werden und Zellen von Typ a) und b) dann **dadurch** identifiziert werden, dass jeder Zelltyp Licht einer typischen und spezifischen Wellenlänge emittiert bzw. absorbiert.

9. Verfahren nach Anspruch 8, **dadurch gekennzeichnet, dass** man einen Hinweis auf die Menge an Cyclin E und Post-G₁₋Substanz in jeder individuellen Zelle erhält, indem man die Lichtintensität oder die Lichtabsorption analysiert, die Zellen entspricht, die auf a) bzw. b) gefärbt sind.

10. Verfahren nach Anspruch 7, **dadurch gekennzeichnet, dass** die Kerne der Zellen identifiziert werden, indem die Probe mit einem Farbstoff gefärbt wird, der für den Zellkern spezifisch ist, wobei der Zellkern bei Bestrahlung Licht einer Wellenlänge emittiert oder alternativ absorbiert, die für den verwendeten Farbstoff spezifisch ist.

11. Verfahren nach Anspruch 10, **dadurch gekennzeichnet, dass** man das von der Probe emittierte oder absorbierte Licht mit einer CCD-Kamera mit einem Filter, der zur Trennung des Lichts von dem Zellkern und des Lichts von den auf a) und b) angefärbten Zellen ausgelegt ist, aufnimmt oder nachweist, Daten zur Lichtintensität jeder Lichtwellenlänge von jedem Zellkern zur Bildanalyse in ein Computerprogramm extrahiert, was eine Messung sowohl des Gehalts an Cyclin E als auch an Post-G₁-Substanz in jedem Zellkern ergibt.

12. Verfahren nach Anspruch 10, **dadurch gekennzeichnet, dass** die Probe durchflusszytometrisch analysiert wird, was den Gehalt an Cyclin E an Post-G₁-Substanz in jedem Zellkern ergibt.

13. Verfahren nach Anspruch 11 oder 12, **dadurch gekennzeichnet, dass** Information, dass eine Probe in der S-Phase und/oder G₂-Phase einen erhöhten Cyclin E-Gehalt aufweist, ein Anzeichen dafür ist, dass die Probe Zellen mit einer gestörten Zelizyklusregulation enthält, was bei Krebserkrankungen von diagnostischem und prognostischem Wert ist.

## Revendications

1. Procédé pour détecter un dérèglement de la régulation du cycle cellulaire dans des cellules individuelles pour diagnostiquer un cancer ou des lésions précancéreuses dans un prélèvement de cellules ou de tissu ou pour pronostiquer une maladie cancéreuse déterminée, **caractérisé en ce qu'**il comprend les étapes consistant à :
détecter de la cycline E dans des cellules distinctes du prélèvement ;
détecter des substances post-G₁ choisies parmi la cycline A, le PCNA et la bromodésoxyuridine dans des cellules distinctes du prélèvement ;
identifier des cellules ayant de la cycline E dans le noyau cellulaire ;
identifier des cellules en phase S ou en phase G₂ en se basant sur leur contenu en substances post-G₁ ; et
moyennant quoi, une quantité élevée de cellules ayant un contenu élevé en cycline E dans le noyau cellulaire alors que ces mêmes cellules sont en phase S ou en phase G₂ indique la présence dans le prélèvement de cellules ayant un dérèglement de la régulation du cycle cellulaire, ce qui a une valeur de diagnostic et de pronostic dans les maladies cancéreuses.

2. Procédé selon la revendication 1, **caractérisé en ce que** la détection de la cycline E est faite par coloration de la protéine.

3. Procédé selon la revendication 2, **caractérisé en ce que** la coloration d'une cycline E choisie est faite par un anticorps dirigé contre la cycline E choisie.

4. Procédé selon la revendication 1, **caractérisé en ce que** la détermination du contenu en substance post-G₁ est faite par coloration de celle-ci.

5. Procédé selon la revendication 4, **caractérisé en ce que** la coloration d'une substance post-G₁ choisie est faite par un anticorps dirigé contre la substance post-G₁ sélectionnée.

6. Procédé selon la revendication 5, **caractérisé en ce que** la cycline A est choisie en tant que substance post-G₁.

7. Procédé selon la revendication 2, **caractérisé en ce que** les cellules qui sont colorées pour :
a) le contenu en cycline E, et
b) le contenu en substance post-G₁
sont colorées en utilisant deux couleurs différentes, l'une spécifique pour a) et l'autre spécifique pour b),

8. Procédé selon la revendication 7, **caractérisé en ce que** les cellules sont éclairées et les cellules de type a) et b) sont ensuite identifiées **en ce que** chaque type de cellule émet ou absorbe respectivement la lumière d'une longueur d'onde typique et spécifique.

9. Procédé selon la revendication 8, **caractérisé en ce qu'**une indication de la quantité de cycline E et de substance post-G₁ dans chaque cellule individuelle est obtenue en analysant l'intensité de la lumière ou l'absorption de la lumière correspondant aux cellules colorées pour a) et b), respectivement.

10. Procédé selon la revendication 7, **caractérisé en ce que** les noyaux cellulaires sont identifiés en colorant le prélèvement avec une couleur qui est spécifique du noyau cellulaire, moyennant quoi le noyau cellulaire quand il est éclairé émet ou bien absorbe de la lumière d'une longueur d'onde spécifique de la couleur utilisée.

11. Procédé selon la revendication 10, **caractérisé en ce que** la lumière émise ou absorbée par le prélèvement est photographiée ou détectée par une caméra à transfert de charge avec un filtre adapté pour séparer la lumière émise par le noyau cellulaire et la lumière émise par les cellules colorées pour a) et b), des informations en rapport avec l'intensité de la lumière de chaque longueur d'onde de lumière émise par chaque noyau cellulaire étant extraites dans un programme informatique d'analyse d'image, donnant une mesure à la fois du contenu en cycline E et en substance post-G₁ dans chaque noyau cellulaire.

12. Procédé selon la revendication 10, **caractérisé en ce que** l'on fait une analyse par cytométrie en flux du prélèvement donnant le contenu en cycline E et en substance post-G₁ de chaque noyau cellulaire.

13. Procédé selon la revendication 11 ou 12, **caractérisé en ce que** l'information selon laquelle un prélèvement contient une quantité élevée de cycline E dans la phase S et/ou la phase G₂ indique que le prélèvement contient des cellules avec un dérèglement de la régulation du cycle cellulaire, ce qui a une valeur de diagnostic et de pronostic dans les maladies cancéreuses.
